(19) Europäisches Patentamt
European Patent Office
Office européen des brevets

(11) Publication number: **0 051 981**
**B1**

(12) # EUROPEAN PATENT SPECIFICATION

(45) Date of publication of patent specification: **26.02.86**

(51) Int. Cl.$^4$: **C 07 D 207/32,** A 61 K 31/40

(21) Application number: **81305251.1**

(22) Date of filing: **04.11.81**

(54) **Esters of N-(4'-hydroxyphenyl)acetamide with 5-benzoyl-1-methylpyrrole-2-acetic acids.**

(30) Priority: **11.11.80 ES 497136**

(43) Date of publication of application:
**19.05.82 Bulletin 82/20**

(45) Publication of the grant of the patent:
**26.02.86 Bulletin 86/09**

(84) Designated Contracting States:
**AT BE CH DE FR GB IT LI LU NL SE**

(56) References cited:
**EP-A-0 002 392**
**US-A-3 865 840**

**CHEMICAL ABSTRACTS, vol. 90, no. 18, April 30, 1979, page 594, abstract 151981e, COLUMBUS, OHIO (US)**
**Houben Weyl-Methoden der Organischen Chemie, Band VIII (1952) page 543**
**The Merck Index (1976) no. 9215, page 1224**
**Remington's Pharmaceutical Sciences (1975) pages 1047-48**
**Burger's medicinal chemistry-Part 1, (1975) pages 178-179**

**The file contains technical information submitted after the application was filed and not included in this specification**

(73) Proprietor: **CALZADA Y CIA, S.R.C.**
**Angel Guimera, 123-125**
**Esplugas De LLobregat Barcelona (ES)**

(72) Inventor: **Calzada Badia, Jose Ma**
**Vilana 11**
**Barcelona 22 (ES)**
Inventor: **Boleda Vila, Antonio**
**Anglí 19-21**
**Barcelona 17 (ES)**
Inventor: **Sabater Sanmartin, Jose**
**Travesera de las Corts, 241**
**Barcelona 28 (ES)**
Inventor: **Villazon Meneses, Ma Jesus**
**Travesers de las Corts, 37**
**Barcelona 28 (ES)**

(74) Representative: **Allard, Susan Joyce et al**
**BOULT, WADE & TENNANT 27 Furnival street**
**London EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to esters of N-(4'-hydroxyphenyl)acetamide with 5-benzoyl-1-methyl-pyrrole-2-acetic acids, which have the following general formula:—

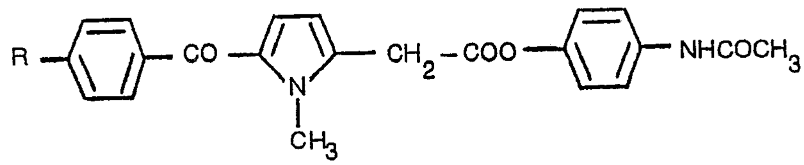

(I)

in which R is a straight or branched chain alkyl group containing from 1 to 4 carbon atoms, or a halogen atom.

The compounds of general formula (I) include all the optically active forms and racemic mixtures thereof.

These compounds possess an anti-inflammatory, analgesic and/or antipyretic activity, and are, therefore, of therepeutic interest. They can be formulated as pharmaceutical compositions for oral or rectal administration, these compositions containing the compounds as active ingredients, together with suitable carriers, diluents or common solvents. They can be administered orally in the form of capsules, tablets, dragees, solutions, suspensions, syrups or emulsions, and rectally, as suppositories. The amount of active ingredients for administration can vary within wide limits, depending on the therapeutic activity.

The compounds described effectively comprise two pharmacologically active parts, of which one is an alcohol, such as N-(4'-hydroxyphenyl) acetamide, with analgesic activity (see Remington's Pharmaceutical Sciences (1975), pages 1047—1048), and the other of which is an acid such as 5-benzoyl-1-methylpyrrole-2-acetic acid, with a strong anti-inflammatory activity (see US—A—3 865 840). When the compounds are administered orally, the medicine is absorbed as such, reaching the blood stream intact, where it is spread through the action of plasmatic stearases, the acid and alcohol components, each performing their own therapeutic function.

Among the compounds which may be obtained by the method object of the present invention, are 4'-acetamidophenyl-2-(5'-*p*-toluyl-1'-methylpyrrole)acetate and 4'-acetamidophenyl-2-(*p*-chlorobenzoyl-1'-methylpyrrole)acetate, which are of the greatest therapeutical interest.

The compounds of the present invention may be obtained from 5-benzoyl-1-methylpyrrole-2-acetic acids, according to a general synthesis method, by the reaction of these acids with a halide or a phosphorus or sulphur oxyhalide, and subsequently this acyl halogenide is reacted with N-(4'-hydroxyphenyl) acetamide, to provide a compound of the following general formula:—

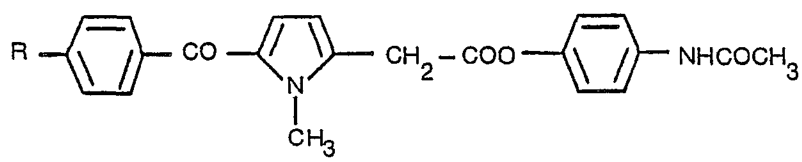

(I)

in which R is as defined above.

The general method for the synthesis of the esters of formula (I), is indicated in the following general reaction scheme:—

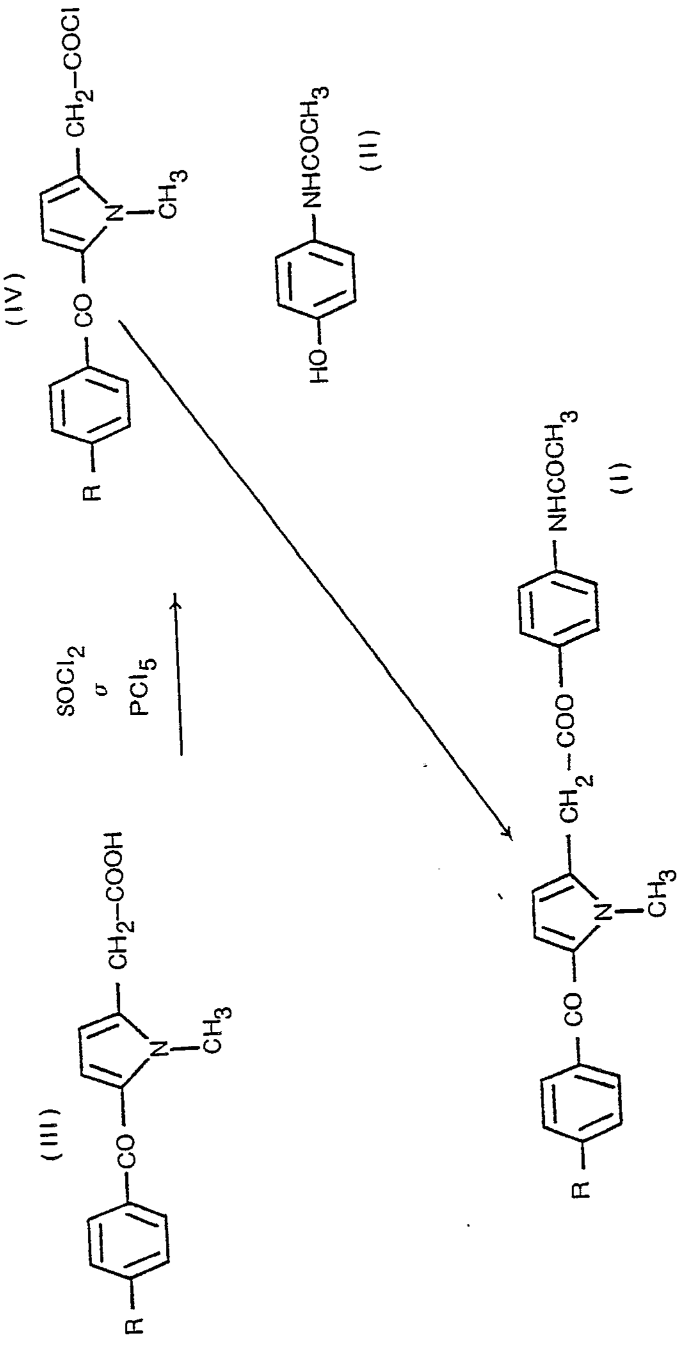


(III)

(IV)

(II)

(I)

Consequently, the general synthesis includes the step of obtaining an acyl halogenide (IV) from a compound of the general form (III) and the subsequent treatment with the alcohol (II) to obtain the compound (I).

The intermediate compound (IV) is obtained from the initial acid form (III), using general acyl halogenide synthesis methods, using either $SOCl_2$ or $PCl_5$ in an anhydrous medium. It can also be obtained from the respective acyl halogenide of (III), making it react in an anhydrous organic solvent medium with $PBr_3$.

Subsequently, the interaction of the acyl halogenide (IV) with the alcohol (II) in a suitable anhydrous organic solvent, provides the desired compound of general formula (I).

To assist in understanding the process for the preparation of the compounds of the present invention, an example is given below of the method used to obtain 4'-acetamidophenyl-2-(5'-*p*-toluyl-1'-methylpyrrole) acetate, following similar methods for obtaining other compounds included in those of general formula (I).

Example

a) Synthesis of 2-(5'-*p*-toluyl-1'-methylpyrrole) acetyl chloride

12 g of 5-*p*-toluyl-1'-methyl-pyrrole-acetic acid was dissolved in 100 ml of chloroform. 5 ml of $SOCl_2$ was added and the mixture kept under reflux for 24 hours. The chloroform was distilled off, 100 ml of toluene added and the mixture then distilled again. The residue was a dark thick oil, which was the acyl chloride, which was used without purifying in the following reaction.

b) Synthesis of 4'-acetamidophenyl-2-(5'-*p*-toluyl-1'-methyl-pyrrole) acetate

The previous residue from step (a) was dissolved in 250 ml of hexane and a solution of 7 g of N-(4'-hydroxyphenyl) acetamide dissolved in 20 ml of pyridine was added thereto. The mixture was shaken at room temperature for 24 hours and the acidified with 1N HCl to pH 2. The desired product was suspended between the aqueous layer and the organic layer. The product was collected by filtration and then washed with hexane. 10 g of product were obtained having a melting point of 210°C. By thin layer chromatography on Kiesegel Merck F 254, using as developing solvent chloroform/acetic acid (19:1), a single stain, was produced whose Rf was 0.43.

The analysis calculated for $C_{23}H_{22}N_2O_4$ was as follows:—

C: 70.77%, H: 5.64%; N: 7.18%; O: 16.41%.

For the compound obtained, the analysis was

C: 70.70%; H: 5.61%; N: 7.00%; O: 16.39%.

**Claims**

1. A compound of the general formula:—

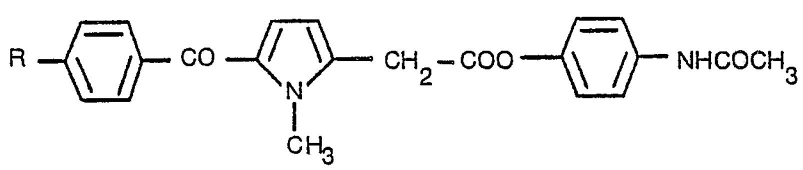

(I)

wherein R is a straight or branched chain alkyl group containing from 1 to 4 carbon atoms, or a halogen atom.

2. 4'-Acetamidophenyl-2-(5'-p-toluyl-1'-methylpyrrole)acetate.

3. 4'-Acetamidophenyl-2-(p-chlorobenzoyl-1'-methylpyrrole)acetate.

4. A pharmaceutical composition which comprises as the active ingredient thereof a compound as claimed in any one of claims 1 to 3 together with an inert diluent or carrier.

5. A compound as claimed in any one of Claims 1 to 3 for use in the treatment of inflammatory conditions.

6. A compound as claimed in any one of Claims 1 to 3 for use in the treatment of pain.

7. A compound as claimed in any one of Claims 1 to 3 for antipyretic use.

**Patentansprüche**

1. Eine Verbindung der allgemeinen Formel

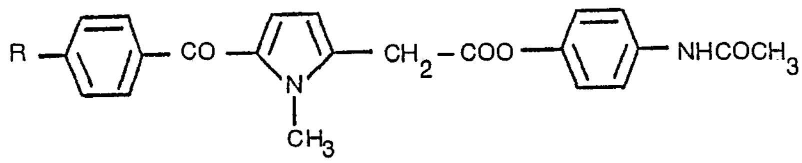

(I)

worin R eine geradkettige oder verzweigtkettige Alkylgruppe mit 1 bis 4 Kohlenstoffatomen oder ein Halogenatom ist.

2. 4'-Acetamidophenyl-2-(5'-p-toluyl-1'-methylpyrrol)acetat.

3. 4'Acetamidophenyl-2-(-p-chlorbenzoyl-1'-methylpyrrol)-acetat.

4. Eine pharmazeutische Zusammensetzung, welche als aktiven Bestandteil derselben eine Verbindung gemäß einem der Ansprüche 1 bis 3 zusammen mit einem inerten Verdünnungsmittel oder Träger enthält.

5. Eine Verbindung nach einem der Ansprüche 1 bis 3 zur Verwendung bei der Behandlung von entzündlichen Zuständen.

6. Eine Verbindung nach einem der Ansprüche 1 bis 3 zur Verwendung bei der Behandlung von Schmerzen.

7. Eine Verbindung nach einem der Ansprüche 1 bis 3 zur Verwendung als Antipyretikum.

**Revendications**

1. Un composé de formule générale

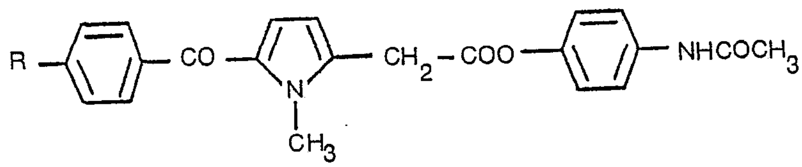

(I)

où R est un groupe alkyle à chaîne droite ou ramifiée contenant d'1 à 4 atomes de carbone, ou un atome d'halogène.

2. Acétate de 4'-acétamidophényl-2-(5'-p-toluyl-1'-méthylpyrrole).

3. Acétate de 4'-acétamidophényl-2-(p-chlorobenzoyl-1'-méthyl-pyrrole).

4. Une composition pharmaceutique qui comprend comme ingrédient actif un composé selon l'une quelconque des revendications 1 à 3, avec un diluant ou substrat inerte.

5. Un composé selon l'une quelconque des revendications 1 à 3, pour utilisation dans le traitement d'affections inflammatoires.

6. Un composé selon l'une quelconque des revendications 1 á 3, pour utilisation dans le traitement de la douleur.

7. Un composé selon l'une quelconque des revendications 1 à 3, pour utilisation antipyrétique.